# EUROPÄISCHE PATENTANMELDUNG

(11) **EP 1 537 869 A2**
(43) Veröffentlichungstag der Anmeldung: **08.06.2005**
(21) Anmeldenummer: 04028545.4
(22) Anmeldetag: 02.12.2004
(51) Int. Cl.: A61K 31/44, A61K 31/415, A61K 31/4164, A61K 33/24, A61P 35/02, C07C 249/02

(54) **Radiosensibilisatoren und Herstellungsverfahren**

(30) Priorität: 04.12.2003 DE 10356567
(71) Anmelder: Christian-Albrechts-Universität zu Kiel, 24098 Kiel (DE)
(72) Erfinder: Krause, Hans-Joachim,Dr., 24161 Kiel (DE)
(74) Vertreter: Biehl, Christian, Dipl.-Phys.

(57) **Zusammenfassung**

Radiosensibilisator mit einem dominierenden α-Diimin-Strukturelement und mindestens einem biologisch aktiven N-Kompartiment.

## Beschreibung

Die Erfindung betrifft besonders elektronenaffine Verbindungen, die als Radiosensibilisatoren in der kombinierten Tumortherapie (Radiochemotherapie) eingesetzt werden können und deren Herstellung.

Es ist bekannt, dass sich neben Sauerstoff zahlreiche organische Verbindungen als Radiosensibilisatoren eignen. Ihnen allen gemeinsam ist ihre große Elektronenaffinität. Folgende Verbindungen resp. Verbindungsklassen werden als Radiosensibilisatoren angesehen und wurden entsprechend untersucht:
1. Nitroverbindungen (z.B. Derivate des 5-Nitroimidazols: Metronidazol, Iso-Metronidazol, Nimorazol; Derivate des 2-Nitroimidazols: Misonidazol, Etanidazol, Pimonidazol, RSU-1069; Chlornitroimidazole, Nitrothiazole, - chinoline, -furane)
2. aromatische Ketone (z.B. Naphthochinone, wie u.a. das Lapachol), Chinone, Chinoline (z.B. das Antioxidantium MTDQ)
3. halogenierte Uridine (z.B. Bromdesoxyuridin)
4. 3-Desoxyadenosin und -guanosin, 3-Aminobenzamide, Dactinomycin bzw. Doxorubicin
5. Anästhetika, Tranquilizer

Ihre Herstellung erfolgt nach bekannten Synthesemethoden der organischen Chemie.

Viele der aufgeführten Verbindungen verursachen aber auch dosislimitierte Nebenwirkungen, u.a. neurotoxische Wirkungen, die nur in Einzelfällen durch Gabe von Phenytoin und Phenobarbital oder auch von Dexamethason und Phenytoin-Na gesenkt werden können. In den letzten Jahren wurde deshalb versucht, ähnliche, aber weniger toxische Verbindungen oder Verbindungen mit einer größeren Elektronenaffinität resp. Verbindungen, die über mehrfunktionelle Mechanismen wirken, zu synthetisieren und als Radiosensitizer zu untersuchen.

An verschiedenen Beispielen wurde nach Literaturangaben gezeigt, dass eine Koordinierung von organischen potenziellen Radiosensibilisatoren an ein Metallzentrum mit bestimmten Redoxpotenzial zu einer deutlichen Zunahme ihrer radiosensibilisierenden Wirkung in hypoxischen Zellen und Abnahme der Toxizität der Ausgangskomponenten führt.

Als typische Vertreter von Metallkomplexen seien genannt:

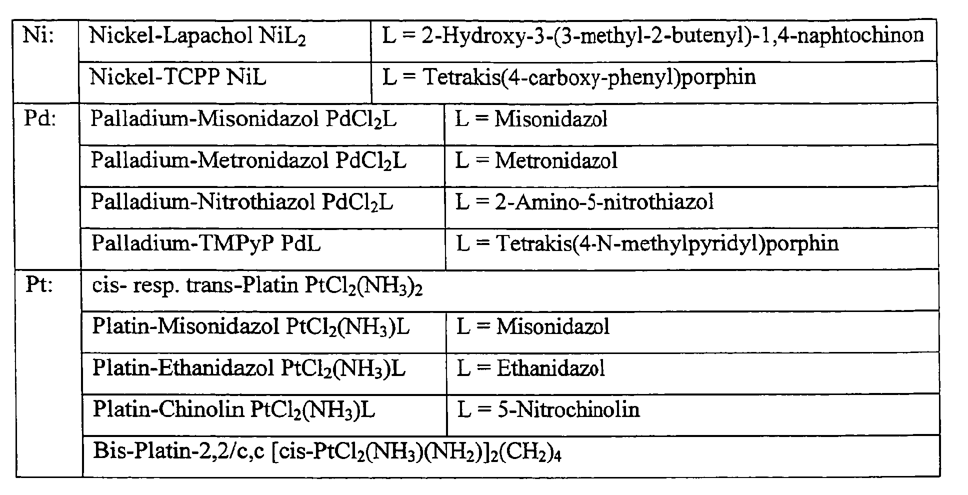

Alle bisher bekannten Verbindungen haben jedoch den Nachteil, dass ihre Darstellung nur in mehrstufigen und aufwendigen Syntheseverfahren erfolgen kann und die in ihnen enthaltenen funktionellen Gruppen ein nicht zu vertretendes Maß an Toxizität gegenüber gesunden euoxischen Zellen besitzen. Weiterhin besitzen viele der genannten Verbindungen einen nur sehr schwachen radiosensibilisierenden Effekt, verbunden mit einem wenig ausgeprägten Elektronenakzeptorvermögen und eine nachteilige geringe Wasserlöslichkeit, was ihre Diffundierungsfähigkeit im Zellgewebe erniedrigt. Darüber hinaus sind Radiosensibilisatoren mit kaum toxischen Strukturelementen, d.h. Strukturelementen über die der notwendige elektronenaffine Charakter relevanter Verbindungen eingestellt werden kann, bislang unbekannt. Als weiteren Nachteil wird die z.T. geringe Stabilität von bisher untersuchten Radiosensibilisatoren angesehen.

Der Erfindung liegt daher die Aufgabe zugrunde, Radiosensibilisatoren zu entwickeln, die einen hohen radiosensibilisierenden Effekt in hypoxischen Zellen und einen relativ hohes Redoxpotenzial bzw. einen einzustellenden elektroaffinen Charakter besitzen. Sie sollten geringe toxische Nebenwirkungen, eine niedrige Lipophilie bei guter Wasserlöslichkeit und eine notwendige DNA-Bindungsfähigkeit mit ausreichender Akkumulationsrate in der Zelle besitzen. Die Synthese der Radiosensibilisatoren soll in einem einzigen Reaktionsschritt mit hoher Ausbeute, mit wenig oder leicht abtrennbaren Nebenprodukten und in Abwesenheit von Metallen oder in Anwesenheit von vorzugsweise Nickel, Palladium oder Platin erfolgen.

Die Aufgabe wird erfindungsgemäß gelöst durch einen Radiosensibilisator mit einem dominierenden α-Diimin-Strukturelement und mindestens einem biologisch aktiven N-Kompartiment.

Der erfindungsgemäße Radiosensibilisator besteht aus neuartigen Diazadienen und Pyridinaldiminen mit verschiedenen heterocyclischen (5- und 6-Ringsysteme mit 1 oder 2 Ringstickstoffatomen), heteroaromatischen (5- und 6-Ringsysteme mit 1 oder 2 Ringstickstoffatomen) und aromatischen Kompartimenten R (auch als N-Substituenten bezeichnet), die wegen ihrer Bioaktivität entweder selber oder als Derivate therapeutische Anwendung finden. Derartige Kompartimente sind Aminderivate, wie z.B. Aminophenazon, Histamin, Tryptamin, Aminoethylpiperazin oder Aminoethylpyrrolidin, in DAD- und PCD-Synthesen.

Die neu dargestellten α-Diimine (R₁-N=C-C=N-R₂) sind farbige, z.T. kristalline, an der Luft kurzzeitig stabile Feststoffe mit definierten Schmelzpunkten. Während die Diazadiene nicht oder nur sehr wenig in H₂O, besser in organischen Lösungsmitteln löslich sind, sind die Pyridinaldimine ausgesprochen gut in Wasser und den üblichen organischen Lösungsmitteln löslich. Alle α-Diimine sind protolyseempfindlich.

Der erfindungsgemäße Radiosensibilisator wurden nach verschiedenen Synthesewegen z.T. in sehr guten Ausbeuten dargestellt und nach den üblichen physiko-chemischen und strukturanalytischen Analysemethoden charakterisiert:

Neben katalytisch gestalteten Kondensationsreaktionen (2 Mole eines entsprechenden primären Amins und 1 Mol einer 1,2-Dicarbonylverbindung für DAD bzw. 1 Mol Amin und 1 Mol Monocarbonylverbindung für PCD) wurde die sog. Umaminierung (Austausch der Stickstoff-Kompartimente eines bestehenden DADs bzw. PCDs durch das gewünschte Stickstoff-Kompartiment eines Amins) für bestimmte, nach der o.g. Methode nicht darstellbaren α-Diimine z.T. lösungsmittelfrei angewandt. In die hier beschriebenen Diiminsynthesen wurden als Carbonylverbindungen Glyoxal, 2,3-Butandion, DL-Campherchinon und Pyridinaldehyd erfolgreich eingesetzt.

Die unter Schutzgas durchgeführten Kondensationsreaktionen wurden katalytisch gestaltet. Als Katalysatoren wurden Ameisensäure in Methanol (im Fall von Glyoxal), p-Toluolsulfonsäure in Benzol (im Fall von 2,3-Butandion und Pyridinaldehyd) sowie Zinkchlorid-Diethylether-Komplex in Dichlormethan und HMPT (im Fall von Campherchinon) eingesetzt. Die notwendige Entfernung des (Reaktions-) Wassers erfolgte durch Trocknungsmittel wie Na₂SO₄ und / oder wasserfreiem CaSO₄ in einer Soxhlethülse.

Der erfindungsgemäße Radiosensibilisator ist gegenüber dem Stand der Technik besonders vorteilhaft. Er besitzt, unter Einsatz biologisch bekannter Verbindungen als N-Kompartimente, einen hohen radiosensibilisierenden Effekt in hypoxischen Zellen, einen einzustellenden elektronenaffinen Charakter bei insgesamt relativ hohem Redoxpotenzial, eine geringe Toxizität gegenüber gesunden euoxischen Zellen, hinreichende Wasserlöslichkeit, eine gute Reaktionszeit mit hohen Ausbeuten an der gewünschten Verbindung in einem Reaktionsschritt und wenig oder keine Nebenprodukte.

Bioaktivitätsuntersuchungen an ausgewählten offenen und halboffenen α-Diiminen und entsprechenden α-Diimin-Komplexen der Nickel-Triade umfassten die Bereiche: Radiosensibiliserung, Zytotoxizität, DNA-Bindungsfähigkeit, Metallakkumulation, Lipophilie und Redoxpotenzial. Einige Ergebnisse dieser Untersuchungen sollen im Folgenden die Vorteile der erfindungsgemäßen Radiosensibilisatoren illustrieren.

Nickel-α-Diimin-Komplexe der Strukturtypen (α-Diimin)NiCl₂ und [(α-Diimin)₂Ni](ClO₄)₂ mit dem α-Diimin Antipy-PCD zeigen gegenüber isosteren Palladium- und Platin-Komplexen erhöhte radiosensibilisierende Wirkungen in hypoxischen CHO-ZeIlen; ER = 1,41 resp. 1,37. Für den unkoordinierten Antipy-PCD-Liganden, mit dem bekannten bioaktiven Molekülteil Phenazon, konnte nur ein sehr geringer strahlensensibilisierender Effekt (ER = 1,13) nachgewiesen werden. Die neutralen α-Diimin-Metall-Halogenid-Komplexe (a) der Nickel-Triade haben gegenüber analogen ionischen Perchlorat-Komplexen (b) eine deutlich höhere radiosensibilisierende Wirkung. Folgende Reihen entsprechender Komplexe - bezogen auf das Zentralmetall - mit abnehmenden o.g. sensibilisierenden Eigenschaften wurden ermittelt: für
(a) Ni > Pd > Pt
(b) Ni > Pt > Pd

Von den untersuchten neuen Platin-α-Diimin-Komplexen ist die fünffach koordinierte (Antipy-PCD)PtCl₂(NH₃)-Verbindung wegen ihres relativ hohen strahlensensibilisierenden Effektes (ER = 1,36) - verglichen mit ER-Literaturdaten für Platinkomplexe - bedeutsam. Eine entsprechende Sensibilisierung aerobischer CHO-ZeIlen durch die hier untersuchten Substanzen wurde in keinem Fall beobachtet.

Die in Toxizitätsuntersuchungen am CHO-Zellsystem *in vitro* eingesetzten neuen α-Diimin-Komplexe der Nickel-Triade (α -Diimin: Antipy-PCD) zeigen eine - im Vergleich zum cis-Platin wesentlich geringere (abgestufte) Zytotoxizität (Toxizitätsparameter: PE), sowohl unter hypoxischen als auch unter aerobischen Bedingungen. Die Komplexierung des α-Diimin-Liganden Antipy-PCD durch Nickel, Palladium und Platin erhöht dessen hypoxische Toxizität (bei 1- bzw. 3-stündiger Einwirkungszeit auf das CHO-Zellsystem); unabhängig vom Strukturtyp.

Die aerobische Toxizität von Antipy-PCD wird dagegen durch Metallierung z.T. merklich erniedrigt [z.B. PE (300 µM, 3 Std.): Antipy-PCD = 0,36; (Antipy-PCD)PdCl₂ = 0,40; (Antipy-PCD)PtCl₂(NH₃) = 0,55 (150 µM)].

Bis(α-Diimin)-Metallkomplexe sind wesentlich toxischer als entsprechende mono(α-Diimin)-Komplexe. Die hypoxische Toxizität nimmt in beiden Komplexgruppen bezogen auf das Zentralmetall - in folgender Reihenfolge ab für:
a) bis(α-Diimin)-Komplexe Pd > Pt > Ni
b) mono(α-Diimin)-Komplexe Pt > Pd > Ni

Die Toxizität (T/C in %) weiterer erstmals dargestellter α-Diimin-Komplexe der Nickel-Triade unterschiedlicher Strukturtypen wurde *in vitro* an der menschlichen Mammakarzinomzell-Linie MDA-MB 231 getestet. Aufgrund der geringen Löslichkeit des Diazadienliganden Antipy-DAD und entsprechender Palladium- und Platinkomplexe im Untersuchungsmedium (Richter's Medium) konnten diese Verbindungen nur in Suspension untersucht werden. Sie weisen im üblichen Konzentrationsbereich von 10⁻⁶ bis 10⁻⁵ mol/l keine zytotoxische Wirkung auf, d.h. sie führen zu keiner Hemmung des Zellwachstums.

Von den Antipy-PCD-Komplexen mit ausreichender Löslichkeit im Untersuchungsmedium wurden bei insgesamt niedriger Zellwachstumshemmung abnehmende Toxizitätswerte für isostere Metallkomplexe der Strukturgruppe [(AntipyPCD)₂M](ClO₄)₂ in der Reihe Pd > Pt > Ni und für die Strukturgruppe (Antipy-PCD)MCl₂ in der Reihe Ni > Pt > Pd ermittelt.

Eine merkliche Toxizität zeigen die α-Diimin-Liganden Urac-PCD und 2-MeO-4-NO₂-Ph-PCD und entsprechende Palladium- und Platinkomplexe (z. B. T/C in % von (2-MeO-4-NO₂-Ph-PCD)PtC₄(COOCH₃)₄ = 61 bei einer Konzentration von 5 × 10⁻⁶ mol/l).

Eine geringere Zytotoxizität (IC₅₀) wurde von den Komplexen (AntipyDAD)M(Ppy₃)₂, M = Pd, Pt und (Antipy-PCD)M(N₃)₂, M = Ni, Pd, Pt in *in vitro* Untersuchungen gegenüber der humanen Darmkarzinomzell-Linie SW 602 und z.T. auch gegenüber SW 1116 nachgewiesen. Die entsprechende Nickelverbindung (Antipy-PCD)Ni(N₃)₂ zeigte von den isosteren Azido-Metallkomplexen die ausgeprägteste Aktivität.

### Beispiel 1

Zunächst wird stellvertretend für ein katalytisch gestaltetes Kondensationsverfahren die Synthese eines metallfreien Radiosensibilisators für Pyridin-2-carbaldimin-Verbindungen (PCD) gewählt:

25 mmol des betreffenden z.B. Antipy-NH₂ Amins wurden zusammen mit 1 mmol p-Toluolsulfonsäure als Katalysator in 110 ml Benzol resp. Ethanol vorgelegt. Bei Raumtemperatur (RT) wurden 25 mmol Pyridin-2-carbaldehyd unter Rühren hinzugetropft und anschließend ca. 2 Std. bei 75 °C gerührt. Als Trocknungsmittel diente Molsieb A3. Die gebildeten Pyridinaldimine fallen beim Abkühlen resp. Einengen der Reaktionsansätze an. Die Rohprodukte wurden abfiltriert, mit kaltem Petrolether gewaschen und aus Petrolether, Benzol bzw. Aceton umkristallisiert. Das folgende Pyridinaldimin wurden synthetisiert:

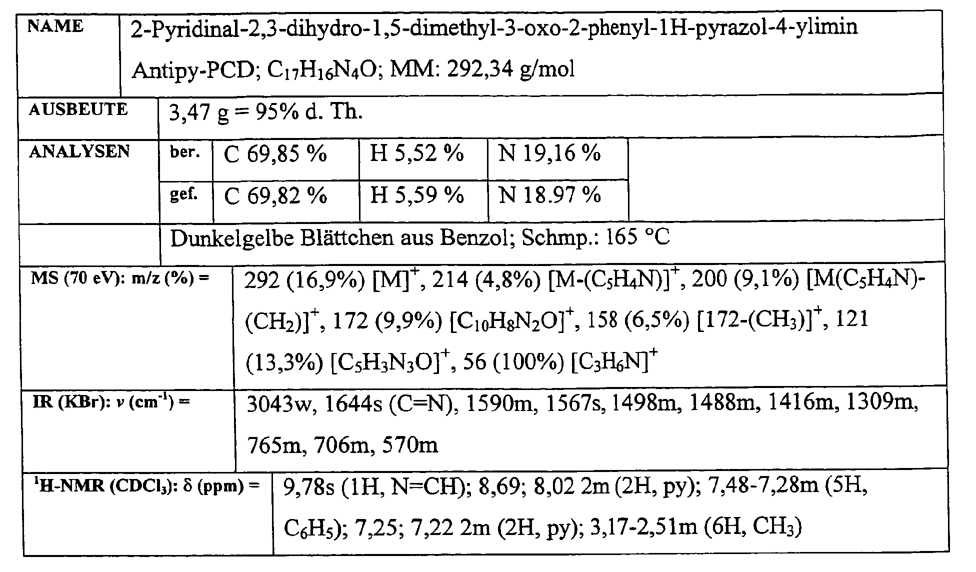

### Beispiel 2

Im Folgenden wird ein Umaminierungsverfahren für die Synthese eines metallfreien Radiosensibilisators am Beispiel von 1,4-Diazabutadien-Verbindungen (DAD) vorgestellt.

30 mmol (5,0 g) t-Bu-DAD und 65 mmol des entsprechenden Amins wurden in einem Reaktionskolben durch Erwärmen ineinander gelöst und 4 Std. bei 40 °C gerührt. Durch Anlegen eines Ölpumpenvakuums bei RT wurde das gebildete t-Butylamin über 2,5 Std. abgezogen und in einer gut gekühlten Vorlage kondensiert. Anschließend wurde der Reaktionskolben im Wasserbad auf ca. 75 °C erwärmt um die Reaktion zu vervollständigen und das restliche Amin zu entfernen. Zu der auf 44 °C abgekühlten nun viskosen, gelblich trüben Schmelze wurden unter Rühren 200 ml eines Lösungsmittelgemisches von Toluol / n-Hexan (1:3) gegeben. Nach 15stündigem Aufbewahren des Reaktionsgefäßes im Kühlschrank wurde das ausgefallene Produkt abgesaugt, mehrmals mit kaltem Hexan gewaschen und aus Ethanol / THF (1 : 1) umkristallisiert.

Nach der genannten Variante wurde erstmals das folgende Diazadien dargestellt:

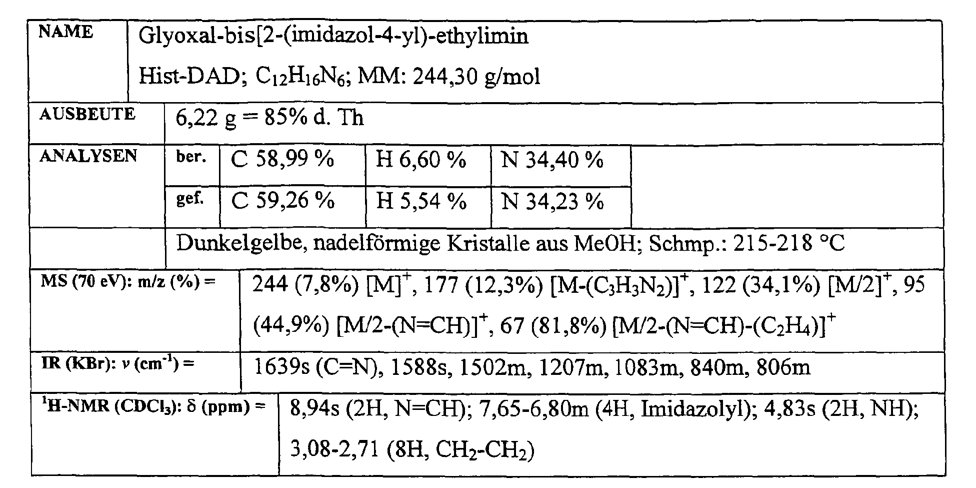

### Beispiel 3

Es folgt die Beschreibung der Synthese eines metallhaltigen Radiosensibilisators am Beispiel eines Nickel-α-Diimin-Komplexes.

5 mmol des Ausgangskomplexes PCDNiCl₂ und 10mmol des entsprechenden PCD-Liganden wurden in 150 ml Ethanol / H₂O (1:1) bei 35 °C gelöst. In die warme Lösung wurden unter Rühren 2,96 g (25,2 mmol) NH₄ClO₄ in 80 ml H₂O innerhalb von 15 min zugetropft. Nach 2-stündigem Rühren bei 50 °C und Abkühlen wurden die ausgefallenen Komplexverbindungen bei RT abfiltriert, mit Diethylether gewaschen und im Vakuum getrocknet. Eine Umkristallisation wurde aus MeOH / Diethylether vorgenommen.

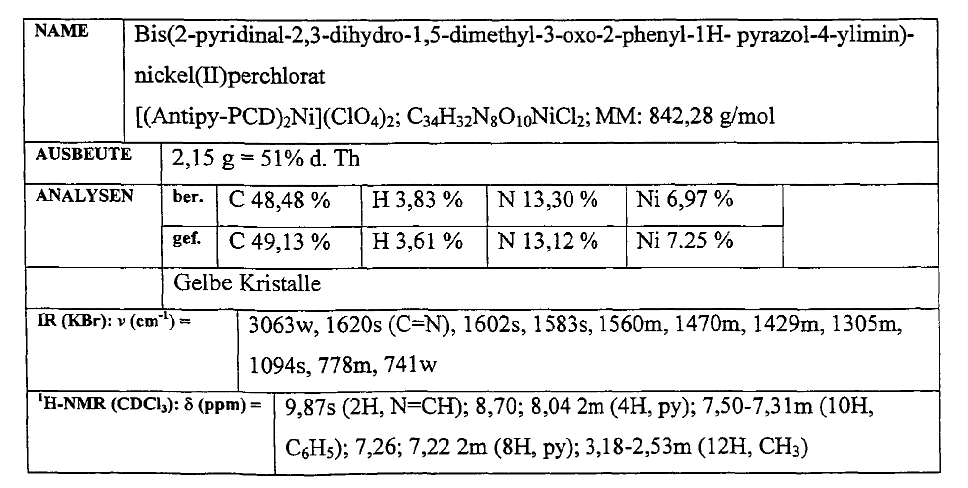

### Beispiel 4

Im Folgenden wird die Synthese eines metallhaltigen Radiosensibilisators als Palladium-α-Diimin-Komplexes am Beispiel von PCDPdCl₂ vorgestellt.

Zu einer Lösung von 2,0 g (7,7 mmol) PdCl₂(CH₃CN)₂ in 400 ml Acetonitril wurden bei 0-2 °C unter Rühren die äquimolare Menge des entsprechenden PCD gegeben. Es wurde 1 h bei dieser Temperatur und 30 min bei RT gerührt. Aus der auf 1/3 des Volumens eingeengten und filtrierten Reaktionslösung kristallisierten bei 4 °C gelbe bis gelborange Nadeln bzw. Blättchen aus, die filtriert, mit Diethylether gewaschen und im Vakuum getrocknet wurden.

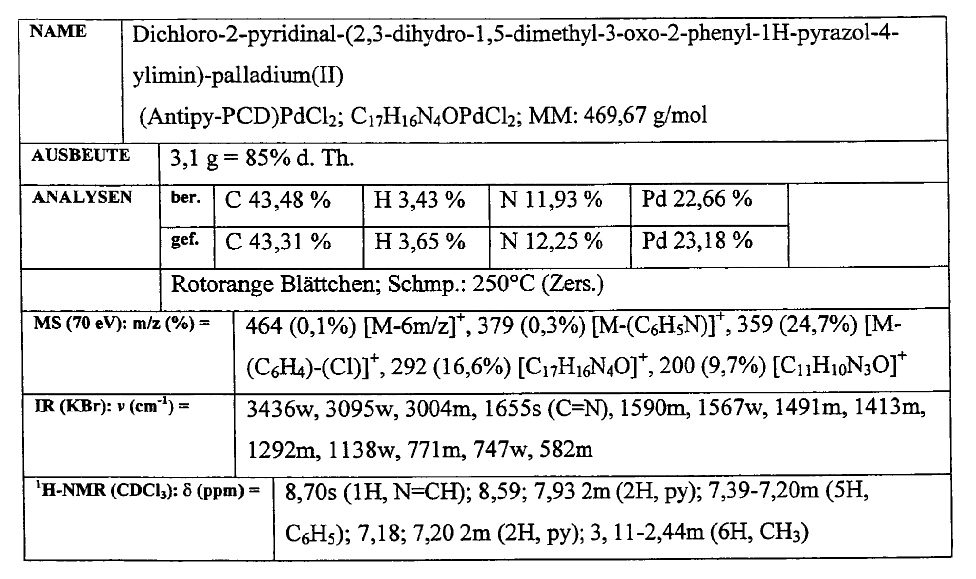

### Beispiel 5

Im diesem Beispiel wird die Synthese eines metallhaltigen Radiosensibilisators am Beispiel eines Platin-α-Diimin-Komplexes exemplarisch für (Antipy-PCD)PtCl₂(NH₃) beschrieben.

In eine auf 10 °C gekühlte Lösung von 5,29 g (5 mmol) Antipy-PCD in 80 ml CH₃OH / H₂O (1:1) wurden unter Rühren 1,8 g (5 mmol) K[PtCl₃(NH₃)] zugegeben. Die Farbe der Reaktionslösung änderte sich von gelb nach orangerot. Es wurde 6 Std. bei RT gerührt. Nach Filtration über Kieselgur wurde die Reaktionslösung im Vakuum bis zur Trockne eingeengt. Der Rückstand wurde in 10 ml CH₃OH gelöst, filtriert, mit 2 ml Et₂O versetzt und auf -5 °C abgekühlt. Die ausgeschiedenen Kristalle wurden abfiltriert, mit gekühltem Et₂O gewaschen und im Vakuum getrocknet.

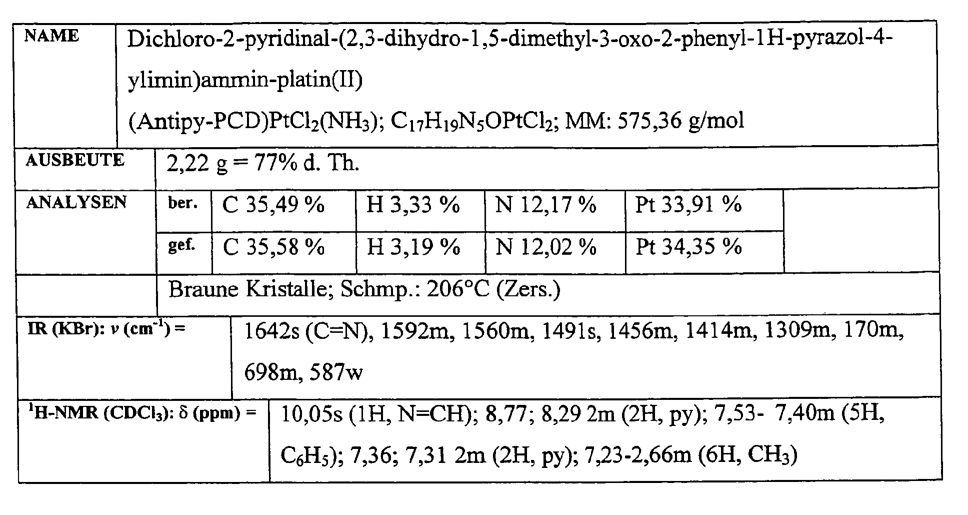

## Patentansprüche

1. Radiosensibilisator,
**gekennzeichnet durch**
ein dominierendes a-Diimin-Strukturelement und
mindestens ein biologisch aktives N-Kompartiment.

2. Radiosensibilisator nach Anspruch 1, **dadurch gekennzeichnet, dass** das mindestens eine biologisch aktive N-Kompartiment ein Aminderivat ist.

3. Radiosensibilisator nach Anspruch 2, **dadurch gekennzeichnet, dass** das Aminderviat Aminophenazon, Histamin, Tryptamin, Aminoethylpiperazin oder Aminoethylpyrrolidin ist.

4. Radiosensibilisator nach einem der vorhergehenden Ansprüche, **dadurch gekennzeichnet, dass** der Radionsensibilisator mit einem Metall komplexiert ist.

5. Verfahren zur Herstellung eines Radiosensibilisators nach einem der Ansprüche 1 bis 3, **dadurch gekennzeichnet, dass** der Radiosensibilisator durch Umaminierungsverfahren dargestellt wird.

6. Verfahren zur Herstellung eines Radiosensibilisators nach einem der Ansprüche 1 bis 3, **dadurch gekennzeichnet, dass** der Radiosensibilisator durch katalytisch gestaltete Kondensationsverfahren dargestellt wird.

7. Verfahren zur Herstellung eines Radiosensibilisators nach Anspruch 6, **dadurch gekennzeichnet, dass** der Katalysator Hexamethylphosphorsäuretriamid ist.

8. Verfahren zur Herstellung eines Radiosensibilisators nach Anspruch 4, **dadurch gekennzeichnet, dass** der Radiosensibilisator durch koordinationschemische Umsetzung dargestellt wird.

9. Verwendung eines Radiosensibilisators nach einem der vorangehenden Ansprüche für therapeutische Zwecke in der Human- und Veterinärmedizin.
